# EUROPÄISCHE PATENTSCHRIFT

(11) **EP 1 673 030 B1**
(45) Veröffentlichungstag und Bekanntmachung des Hinweises auf die Patenterteilung: **09.04.2008**
(21) Anmeldenummer: 05731906.3
(22) Anmeldetag: 19.04.2005
(51) Int. Cl.: A61F 2/00

(54) **FLÄCHIGES NETZIMPLANTAT ZUR HERNIENVERSORGUNG**
PLANAR MESH IMPLANT FOR SUPPLYING HERNIA
FILET PROTHETIQUE PLAT POUR LA REPARATION DE HERNIES

(30) Priorität: 26.04.2004 DE 102004020469
(43) Veröffentlichungstag der Anmeldung: 28.06.2006
(73) Patentinhaber: GfE Medizintechnik GmbH, 90431 Nürnberg (DE)
(72) Erfinder: KÖCKERLING, Ferdinand, 30177 Hannover (DE); ZIMMERMANN, Hanngörg, 91327 Gössweinstein (DE); HEINLEIN, Markus, 91327 Gössweinstein (DE)
(74) Vertreter: Hübner, Gerd
(86) Internationale Anmeldenummer: PCT/EP2005/004133
(87) Internationale Veröffentlichungsnummer: WO 2005/102209

(56) Entgegenhaltungen:
- EP-A- 0 986 993
- DE-A1- 19 945 299
- US-A1- 2003 171 823
- US-A1- 2003 212 460
- US-B1- 6 174 320

## Beschreibung

Die Erfindung betrifft ein flächiges Netzimplantat zur Versorgung von insbesondere Leistenhernien.

Derartige Netzimplantate sind in unterschiedlichsten Konfigurationen in der Medizintechnik üblich und Standardprodukte für die Hernienversorgung. Eine spezielle Ausgestaltung ist beispielsweise der WO 00/67663 A1 zu entnehmen.

Herniennetze werden bei der operativen Reparation von insbesondere Leistenhernien verwendet, um ein spannungsfreies Überdecken des Defektes zur Stabilisierung der Bauchwand zu erzielen. Je nach Art und Lage der Hernien kann es dabei notwendig sein, eine Körperröhre - wie beispielsweise den Samenstrang bei einer Leistenhernie - durch das Implantat zu führen. Hierzu ist in der Netzlage eine Durchlassöffnung angelegt. Da die Körperröhre naturgemäß keinen in die Durchlassöffnung entfädelbaren Anfang besitzt, muss in der Netzlage ein Einführungsschlitz zwischen ihrem äußeren Umriss und der zentralen Durchlassöffnung zum Einführen der Körperröhre dorthin angelegt sein.

In der herkömmlichen Operationstechnik wird dieser Schlitz nach dem Einführen der Körperröhre in die Durchlassöffnung geschlossen, indem die Schlitzflanken in eine Überlapp-Stellung gebracht und miteinander vernäht werden. Dieses Zusammenziehen sorgt allerdings für eine Deformation des Netzimplantates, was eine saubere Lage an der Bauchwand beeinträchtigen kann. Ferner sind derartige Netzimplantate auf Grund der Verwendung von sehr dünnen und leichten Fäden mechanisch nicht sehr stabil, was zu einer schwierigen Handhabbarkeit während der Operation führt.

Die US-A-20030171823 offenbart die Bildung des flächigen Netzimplantates aus zwei ringförmigen, eine zentrale Öffnung umgebenden Netzlagen, die jeweils einen den Ringverlauf unterbrechenden Zugangsschlitz zu ihrer zentralen Öffnung aufweisen. Die beiden Netzlagen sind dabei mit im Wesentlichen fluchtenden zentralen Öffnungen mit versetzt zueinander angeordneten Positionen der Zugangsschlitze aufeinandergelegt und bezogen auf die Peripherrichtung nur auf einer gemeinsamen Seite der Zugangsschlitze miteinander fest verbunden.

Durch die Doppellagigkeit des Netzimplantates mit einer Verbindung zwischen den beiden Lagen ergibt sich zwar einerseits eine verbesserte Stabilität des Netzimplantates, was insbesondere einer problemlosen Ausbreitbarkeit des Implantates an seinem Implantationsort beispielsweise zwischen der Fascie und der Bauchwand zu gute kommt. Die durch das Netzimplantat zu legende Körperröhre ist andererseits durch einfaches Auseinanderziehen der unverbundenen Ringsektoren der beiden Netzlagen und Einschieben in deren zentrale Öffnung dorthin einführbar.

Die Grundform dieses Netzimplantates ist rotations-unsymmetrisch und kann am besten mit "kartoffelförmig" umschrieben werden. Die zentrale Öffnung liegt dabei bezüglich des Schwerpunktes dieser rotationsunsymmetrischen Form exzentrisch. Der Versatzwinkel der beiden Zugangsschlitze beträgt unter 90 °. In der Beschreibung der US-A-20030171823 wird in diesem Zusammenhang zwar als Obergrenze des Versatzwinkels ein Wert von 180 ° genannt. Dies muss jedoch als sehr nachteilig angesehen werden, weil dann der Samenleiter des Patienten von den beiden Schmalseiten her in die Zentralöffnungen eingefädelt werden muss, was zumindest auf einer Seite aufgrund der exzentrischen Anordnung der Zentralöffnungen zu einem sehr langen "Einfädelweg" führt.

Als weiterer Nachteil bei dem Netzimplantat gemäß US-A-20030171823 ist festzuhalten, dass die beiden Netzlagen aufgrund der unsymmetrischen Konfiguration des Netzimplantats unterschiedlich ausgebildet sein müssen. Insoweit sind für die Herstellung des Netzimplantates also unterschiedlich geformte Netzlagen herzustellen, zu bevorraten und zu verarbeiten. Dies stellt einen unerwünschten Mehraufwand dar.

Davon ausgehend liegt der Erfindung die Aufgabe zu Grunde, ein flächiges Netzimplantat zur Hernienversorgung so auszugestalten, dass es bei der Implantation in operationstechnischer Hinsicht nach wie vor sauber zu verlegen ist, wobei das Netzimplantat allerdings aufgrund des Zuschnitts seiner Netzlagen einfacher herzustellen ist.

Diese Aufgabe wird durch die im Kennzeichnungsteil des Anspruches 1 angegebenen Merkmale gelöst. Kern der Erfindung ist die Formgebung der Netzlagen, die identisch ausgebildet sind und eine deckungsgleiche Form besitzen.

Bevorzugte Ausführungsformen, weitere Merkmale, Einzelheiten und Vorteile der Erfindung sind den Unteransprüchen und der nachfolgenden Beschreibung entnehmbar, in der ein Ausführungsbeispiel des Erfindungsgegenstandes an Hand der beigefügten Zeichnungen näher erläutert wird. Es zeigen:
- Fig. 1: eine Draufsicht auf die beiden Netzlagen eines Netzimplantates nach Art einer Explosionsdarstellung, und
- Fig. 2: eine Draufsicht auf das flächige Netzimplantat.

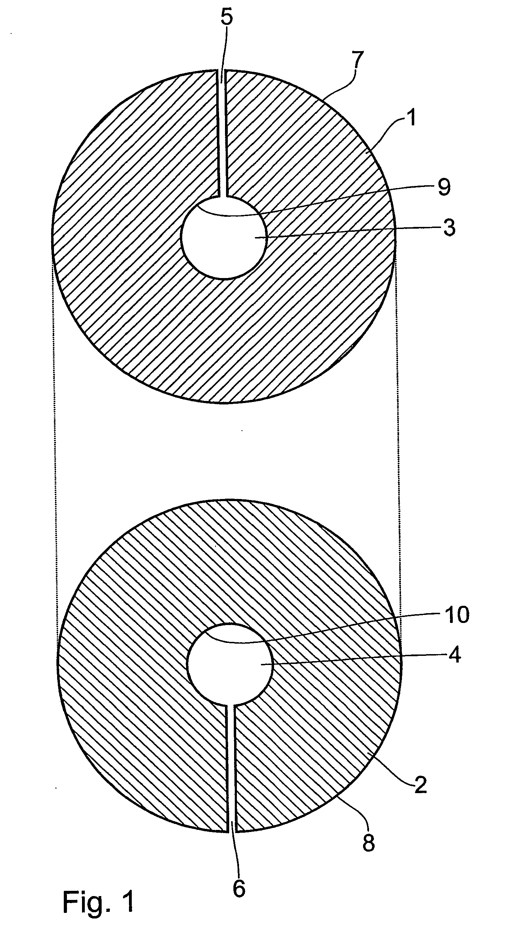

Wie insbesondere aus Fig. 1 deutlich wird besteht das Netzimplantat aus einer ersten und zweiten Netzlage 1, 2, die als identisch ausgebildete Ringe mit einer zentralen Öffnung 3, 4 ausgebildet sind. In radialer Richtung sind beide Netzlagen.1, 2 jeweils mit einem Zugangsschlitz 5, 6 von ihrer peripheren Außenkante 7, 8 zur zentralen Öffnung 3, 4 hin versehen.

Die beiden Netzlagen 1, 2 bestehen aus einem Polypropylen-Monofilament-Netzmaterial, das mit einer Fadenstärke von 100 dtex in Atlas-Legung kettengewirkt ist. Das Flächengewicht dieses Lagenmaterials für jede Netzlage 1, 2 beträgt ca. 60 bis 65 g/m², kann allerdings auch deutlich darunter gewählt werden. Die Netzlagen 1 sind - wie nicht eigens dargestellt ist - mittels Laserschneiden aus einem entsprechenden Bahnmaterial heraus geschnitten.

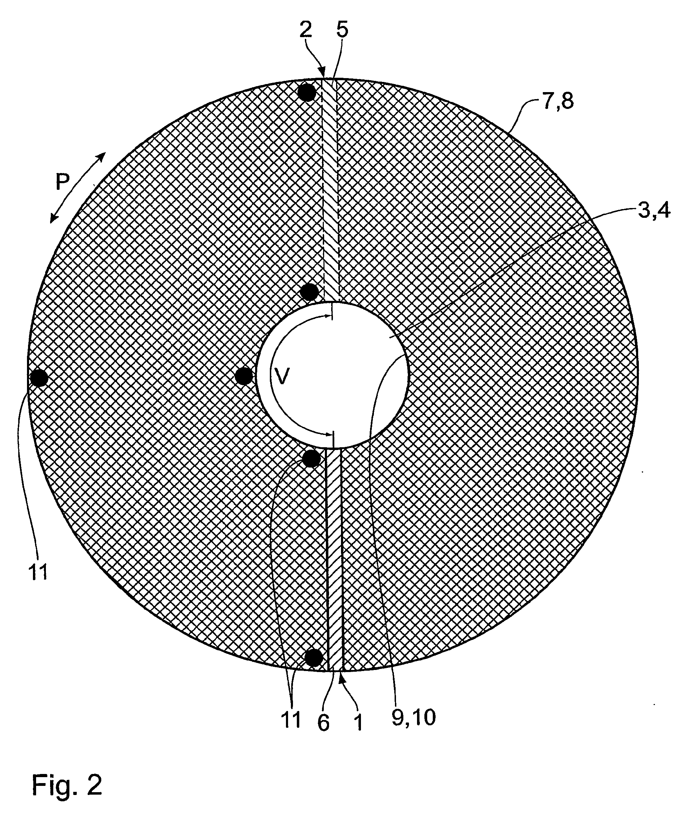

Zur Herstellung des kompletten Netzimplantates, wie es in Fig. 2 in Draufsicht gezeigt ist, werden die beiden so vorkonfektionierten Netzlagen 1, 2 bezüglich ihrer Umrisse deckungsgleich aufeinandergelegt, wobei die beiden Zugangsschlitze 5, 6 jedoch um einen Versatzwinkel V von 180° versetzt zueinander angeordnet sind. Zu einer Seite bezogen auf die Peripherrichtung P der Netzlagen 1, 2 hin - nämlich auf der bezogen auf Fig. 2 linken gemeinsamen Seite der Zugangsschlitze 5, 6 - sind die beiden Netzlagen 1, 2 an drei gleichmäßig über die inneren Umfangskanten 9, 10 der zentralen Öffnungen 3, 4 bzw. Außenkanten 7, 8 verteilte Punktverbindungen 11 miteinander fest verbunden. Die Punktverbindungen 11 können beispielsweise aus Punktnähten, hergestellt aus dem gleichen Fadenmaterial wie die Netzlagen 1, 2 selbst, oder aus Punktverklebungen beispielsweise aus einem thermoplastischen Klebstoff bestehen.

Wie den Zeichnungen ebenfalls nicht explizit entnehmbar ist, ist das Netzimplantat nach seiner Konfektionierung aus den beiden Netzlagen 1, 2 mittels eines aus dem Stand der Technik bekannten PACVD-Prozesses, wie er etwa in der DE 199 45 299 A näher beschrieben ist, mit einer die gesamte. Oberfläche der Filamente bedeckenden Titanisierung als körperverträgliche Beschichtung versehen. Deren Dicke liegt im Bereich von < 2 µm, vorzugsweise bei etwa 5 bis 700 nm.

Diese durchgehende Metallisierungsschicht auf dem Kunststoff-Netzmaterial verbessert erheblich die Gewebeverträglichkeit des Netzimplantates. Diese wird auch durch das bereits erwähnte Laserschneiden der Netzlagen 1, 2 unterstützt, da damit an den Schnittkanten keine "Ausfransungen" mit sich lösenden Faserpartikeln hervorgerufen, sondern ein sauber abgeschmolzener Randkantenbereich erzielt werden.

## Patentansprüche

1. Flächiges Netzimplantat zur Hernienversorgung, umfassend
- eine erste ringförmige, eine zentrale Öffnung (3) umgebende Netzlage (1) mit einem den Ringverlauf unterbrechenden Zugangsschlitz (5) zur zentralen Öffnung (3),
- eine zweite ringförmige, eine zentrale Öffnung (4) umgebende Netzlage (2) ebenfalls mit einem den Ringverlauf unterbrechenden Zugangsschlitz (6) zur zentralen Öffnung (4), wobei
- die beiden Netzlagen (1, 2) mit fluchtenden zentralen Öffnungen (3, 4) bei versetzt zueinander angeordneten Positionen der Zugangsschlitze (5, 6) aufeinander gelegt und bezogen auf die Peripherrichtung (P) nur auf einer gemeinsamen Seite der Zugangsschlitze (5, 6) miteinander verbunden sind, wobei die beiden Zugangsschlitze (5, 6) um einen Versatzwinkel (V) von 180° zueinander versetzt angeordnet sind,
**dadurch gekennzeichnet, dass** die beiden Netzlagen (1, 2) identisch ausgebildet sind und eine deckungsgleiche Form besitzen.

2. Netzimplantat nach Anspruch 1, **dadurch gekennzeichnet, dass** die beiden Netzlagen durch Punktverbindungen (11) in Form von Punktnähten oder -verklebungen miteinander verbunden sind.

3. Netzimplantat nach Anspruch 2, **dadurch gekennzeichnet, dass** das die Punktverbindungen (11) jeweils entlang der inneren Umfangskante (9, 10) der zentralen Öffnung (3, 4) und entlang der Außenkante (7, 8) der Netzlagen (1, 2) angeordnet sind.

4. Netzimplantat nach einem der vorgenannten Ansprüche, **dadurch gekennzeichnet, dass** es aus einem Netz-Bahnmaterial vorzugsweise aus Polypropylen mit Hilfe eines Laser-Schneidstrahls zugeschnitten ist.

5. Netzimplantat nach einem der vorgenannten Ansprüche, **gekennzeichnet durch** eine metallhaltige, durchgehende, körperverträgliche Beschichtung.

6. Netzimplantat nach Anspruch 5, **dadurch gekennzeichnet, dass** die Beschichtung eine Titan-haltige Beschichtung mit einer Dicke von kleiner 2 µm, vorzugsweise von 5 bis 700 nm ist.

## Claims

1. Two-dimensional mesh implant for hernia care comprising
- a first annular mesh layer (1), surrounding a central opening (3), with an access slit (5), interrupting the annular path, towards the central opening (3);
- a second annular mesh layer (2) surrounding a central opening (4), also with an access slit (6) interrupting the annular path, towards the central opening (4);
- with the two mesh layers (1, 2) being superimposed with aligning central openings (2, 4) with the positions of the access slits (5, 6) being offset with respect to one another and the two mesh layers being joined together only on one common side of the access slits (5, 6) based on the peripheral direction (P);
- with the two access slits (5, 6) being positioned offset with respect to one another by an angle (V) of 180°,
**characterised in that** the two mesh layers (1, 2) are of identical design and have a congruent shape.

2. Mesh implant according to claim 1, **characterised in that** the two mesh layers are joined together by connection points (11) in the form of seamed points or bonded points.

3. Mesh implant according to claim 2, **characterised in that** the connection points (11) are in each case positioned along the inner circumferential edge (9, 10) of the central opening (3, 4) and along the outer edge (7, 8) of the mesh layers (1, 2).

4. Mesh implant according to any one of the preceding claims, **characterised in that** it is cut out of a mesh web material, preferably made from polypropylene, using a laser cutting beam.

5. Mesh implant according to any one of the preceding claims, **characterised by** a metal-containing, continuous, body-compatible coating containing metal.

6. Mesh implant according to claim 5, **characterised in that** the coating is a titanium-containing coating with a thickness of less than 2 µm, preferably from 5 to 700 nm.

## Revendications

1. Filet prothétique pour la préparation de hernie, comportant
- un support à filet (1) circulaire, encerclant une ouverture centrale (3), avec une fente d'accès (5) ouvrant le cercle pour l'ouverture centrale (3),
- un second support à filet (2) circulaire, encerclant une ouverture centrale (4), avec lui aussi une fente d'accès (6) ouvrant le cercle pour l'ouverture centrale (4), où
- les deux supports à filet (1, 2) sont posés l'un sur l'autre lorsque les fentes d'accès (5, 6) sont disposées en alternance, et, relativement au sens périphérique (P), reliés l'un à l'autre sur un seul côté commun des fentes d'accès (5, 6), où les deux fentes d'accès (5, 6) sont disposées en alternance selon un angle d'alternance (V) de 180°,
**caractérisé en ce que** les deux supports à filet (1, 2) sont de conception identique et possèdent une forme de couverture similaire.

2. Filet prothéique selon la revendication 1, **caractérisé en ce que** les deux supports à filet sont reliés l'un à l'autre par des points d'assemblage (11) consistant en des points de couture ou de collage.

3. Filet prothéique selon la revendication 2, **caractérisé en ce que** les points d'assemblage (11) sont chaque fois disposés le long de l'arête intérieure de circonférence (9, 10) de l'ouverture centrale (3, 4) et le long de l'arête extérieure (7, 8) des supports à filet (1, 2).

4. Filet prothéique selon l'une des revendications précédentes, **caractérisé en ce qu'**il est découpé dans une feuille de filet et de préférence dans du polypropylène à l'aide d'un rayon laser de découpe.

5. Filet prothéique selon l'une des revendications précédentes, **caractérisé par** une couche métallique continue, que le corps accepte.

6. Filet prothéique selon la revendication 5, **caractérisé en ce que** la couche est une couche contenant du titane avec une épaisseur de moins de 2 µm, de préférence de 5 à 700 nm.
